# EUROPÄISCHE PATENTSCHRIFT

(11) **EP 1 404 710 B1**
(45) Veröffentlichungstag und Bekanntmachung des Hinweises auf die Patenterteilung: **15.06.2011**
(21) Anmeldenummer: 02751064.3
(22) Anmeldetag: 24.06.2002
(51) Int. Cl.: C07K 14/435

(54) **IDENTIFIZIERUNG VON SES-3 (SPR-5) AUS C. ELEGANS SOWIE DESSEN VERWENDUNGEN**
IDENTIFICATION OF SES-3 (SPR-5) IN C. ELEGANS AND THE USES OF THE SAME
IDENTIFICATION DE SES-3 DE C. ELEGANS ET LEURS UTILISATIONS

(30) Priorität: 22.06.2001 DE 10130247
(43) Veröffentlichungstag der Anmeldung: 07.04.2004
(73) Patentinhaber: Sanofi-Aventis Deutschland GmbH, 65929 Frankfurt am Main (DE)
(72) Erfinder: BAUMEISTER, Ralf, 82194 Gröbenzell (DE); LAKOWSKI, Bernard, 81375 München (DE); EIMER, Stefan, 81375 München (DE)
(86) Internationale Anmeldenummer: PCT/EP2002/006964
(87) Internationale Veröffentlichungsnummer: WO 2003/000717

(56) Entgegenhaltungen:
- WO-A-97/11956
- DATABASE EMBL [Online] 20. Juni 2001 (2001-06-20) "C. elegans cosmid Y40B1B" retrieved from EBI Database accession no. AL032636 XP002247708
- WEN C. ET AL. : "spr-2, a suppressor of the egg-laying defect caused by loss of sel-12 presenilin in Caenorhabditis elegans, is a member of the SET protein subfamily" PROC. NATL. ACAD. SCI. (USA), Bd. 97, Nr. 26, 19. Dezember 2000 (2000-12-19), Seiten 14524-14529, XP002247707
- NAGASE T ET AL: "PREDICTION OF THE CODING SEQUENCES OF UNIDENTIFIED HUMAN GENES. IX. THE COMPLETE SEQUENCES OF 100 NEW CDNA CLONES FROM BRAIN WHICH CAN CODE FOR LARGE PROTEINS IN VITRO" DNA RESEARCH, UNIVERSAL ACADEMY PRESS, JP, Bd. 5, Nr. 5, 1998, Seiten 31-39, XP000878819 ISSN: 1340-2838
- DATABASE EMBL [Online] 9. November 2000 (2000-11-09) "C. elegans cosmid T08D10" retrieved from EBI Database accession no. Z50756 XP002247709
- DATABASE EMBL [Online] 1. Juni 2001 (2001-06-01) "D. melamanogaster" retrieved from EBI Database accession no. AE003514 XP002247710
- EIMER S. ET AL.: "Loss of spr-5 bypasses the requirement for the C. elegans presenilin sel-12 by derepressing hop-1" THE EMBO JOURNAL, Bd. 21, Nr. 21, 1. November 2002 (2002-11-01), Seiten 5787-5796, XP002247754

## Beschreibung

Die vorliegende Erfindung betrifft transgene Organismen, welche isolierte Nukleinsäuremoleküle, die für *ses-3* bzw. mutierte ses-3 codieren, enthalten, Verwendungen solcher Nukleinsäuremoleküle zur Erzeugung von Modellorganismen und die Verwendung von transgenen Organismen in Verfahren zur Identifizierung von Substanzen, welche die ses-3 Aktivität verändern, resp. die Presenilin Aktivität (z.B. die von sel-12 oder hop-1) steigern.

### Hintergrund der Erfindung

Die Alzheimer' sche Krankheit ist neben der Parkinsonschen Krankheit die bekannteste neurodegenerative Erkrankung. Das charakteristische Merkmal der Alzheimer'schen Erkrankung ist die Entwicklung neuronaler Proteinaggregationen, sogenannter Plaques, die im wesentlichen aus einem unlöslichen, 4 kDa großen Peptid namens Amyloid Beta-Peptide (Aß4) bestehen. Untersuchungen der letzten Jahre deuten darauf hin, dass die Bildung von Aß4 ursächlich an der Entstehung der Krankheit beteiligt ist. Dominante Mutationen in drei Genen verursachen familiäre Formen der Erkrankung (FAD Familial Alzheimer's Disease). Es wurde gefunden, dass eines der betroffenen Gene für das Amyloid Precursor Protein (APP) kodiert, das durch drei Proteasen prozessiert werden kann, wobei unter anderem Aß4 entsteht. FAD Mutationen in APP erhöhen die Produktionsmenge von Aß42, einer 42 Aminosäure langen Variante von Aß4. Molekulargenetische Untersuchungen von Familien, in denen die Alzheimer'sche Krankheit auftrat, haben zu der Identifizierung der humanen Gene Presenilin 1 und Presenilin 2 geführt (Rogaev et al. 1995, Nature 376, 775-778; Levy-Lahad, E. et al. 1995, Science 269: 973-977), die, durch bestimmte Mutationen verändert, ursächlich am Ausbruch der Alzheimer'schen Krankheit beteiligt sind und welche außerdem während der Entwicklung eine Schlüsselrolle in dem Notch-Signaltransduktionsweg spielen. Presenilinproteine sind ER-Golgi lokalisiert und besitzen mindestens 6 Transmembrandomänen. Es wurde gefunden, dass Mutationen in PS1 und PS2 die Bildung von Aß4 beeinflussen und an der Verursachung der Alzheimer'schen Erkrankung beteiligt sind, vermutlich über die Entstehung von Amyloidablagerungen. Mitglieder der Presenilinfamilie wurden unter anderem in Maus, *Drosophila melanogaster, Xenopus laevis* und im Fadenwurm Caenorhabditis *elegans* identifiziert. Obwohl mutierte Preseniline die Prozessierung von APP im Menschen beeinflussen, ist ihre natürliche Funktion dort nicht detailliert bekannt; eine Funktion als APP-Protease (gamma-Sekretase) wurde postuliert. Aus weiteren Untersuchungen mit menschlichen Zellkulturen ergaben sich Hinweise darauf, dass Preseniline bei der intrazellulären Proteolyese von Notch-ähnlichen Rezeptoren nach deren Aktivierung durch Ligandenbindung eine Rolle spielen. Daher wird angenommen, dass die Preseniline bei der Prozessierung von mindestens zwei unterschiedlichen Membranproteinen (APP und Notch) beteiligt sind. Es wird gegenwärtig kontrovers diskutiert, ob die Preseniline selbst die Proteasen in dieser Prozessierung sind, oder ob sie Kofaktoren dieser Reaktionen sind oder die Proteolyse indirekt beeinflussen (Haass, C. et al. 1999, Science 286 (5441): 916-919).

Ausgehend von den erwähnten Presenilin-Genen wird intensiv geforscht, um die molekularen Erkrankungsprozesse aufzuklären und um Arzneimittel für die Behandlung und Prävention der Alzheimer'schen Krankheit zu entwickeln. Insbesondere wird dabei nach Faktoren gesucht, die die Aktivität der Presenilin-Gene beeinflussen. Dabei kommt der Suche nach Suppressoren des Presenilin-Mutanten Phänotyps eine besondere Bedeutung zu. Suppressoren können Mutationen in anderen Genen sein, die die Notwendigkeit der Presenilinfunktion eliminieren, oder es können Substanzen sein, die die Aktivität anderer Gene oder der Preseniline modulieren, so dass der Presenilin-Mutanten-Defekt keine phänotypischen Auswirkungen hat.

Als ein bevorzugter Modellorganismus wurde bei solchen Untersuchungen vielfach ein Nematode, insbesondere *Caenorhabditis elegans* eingesetzt. Der Vorteil eines Nematodenmodells, insbesondere des *C. elegans*-Modells, besteht vor allem in der Eignung für ein Hochdurchsatzverfahren (HTS; High-Throughput-Screening), der Möglichkeit einer schnelleren genetischen Analyse durch geringere Generationszeit (2-3 Tage) und einem detaillierterem Wissen über molekulare und funktionelle Eigenschaften des Nervensystems in *C*. *elegans.* Da *C*. *elegans* in Mikrotiterplatten gehalten werden kann, können mit diesem Testsystem 10.000 und mehr Substanzen in kurzer Zeit per HTS am lebenden Wurm ausgetestet werden.

Im Fadenwurm C. *elegans* wurden bisher drei Presenilingene namens *sel-12, hop-1* und *spe-4* identifiziert. Spe-4 ist das divergenteste Mitglied der Familie. Mutationen in spe-4 führen zu einem Defekt der cytoplasmatischen Partitionierung während der Spermatogenese (L'Hernault, S. W. et al. 1992, J Cell Biol 119: 55-68). Mutationen in hop-1 haben keinen sichtbaren Phänotyp, führen aber in Kombination mit Mutationen in sel-12 zu einem synthetisch letalen Phänotyp (Li, X. et al. 1997, Proc Natl Acad Sci USA 94: 12204-12209). Sel-12 ähnelt den menschlichen PS1 und PS2 am stärksten und ist auch das am besten studierte Presenilingen in *C*. *elegans.* Es wurde gefunden, daß bestimmte sel-12 Mutanten einen Eiablagedefekt aufweisen und morphologische Veränderungen der Vulva-Entwicklung zeigen (Levitan, D. et al. 1995, Nature 377: 351-4). *Sel-12* ist 50 % identisch zu humanen Presenilinen und der Eiablagedefekt von *sel-12* Mutanten lässt sich durch transgene Expression von PS1 oder PS2 vom *sel-12* Promoter aus kompensieren (Baumeister et al., 1997, Genes and Function 1: 149-159). Dies zeigt, dass PS1 und PS2 funktionelle Homologie zu *sel-12* aufweisen. *Sel-12* Mutationen wurden zuerst als Suppressoren des Multivulva Phänotyps einer Funktionsgewinnmutante (gain-of-function) im *lin-12* und *glp-1* Notch Rezeptor-Gen identifiziert. Notch Rezeptoren kontrollieren die Determinierung von Zellschicksalen in vielen mehrzelligen Organismen. Außerdem wurde gezeigt, dass der Phänotyp von *sel-12* Mutanten dem von schwachen Funktionsverlust-Mutanten von *lin-12* ähnelt. Zusammenfassend lässt sich feststellen, dass Preseniline bei der Notch Signaltransduktion in zahlreichen Organismen, einschließlich den Säugern, eine wichtige Rolle spielen.

### Zusammenfassung der Erfindung

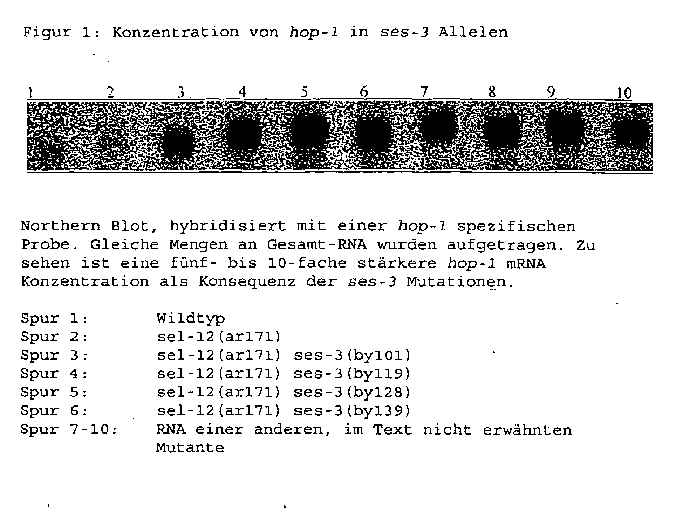

Die gegenwärtigen Erfinder haben nun überraschend festgestellt, dass bestimmte Mutationen in einem weiteren Gen, dem von den Erfindern erstmals identifizierten *ses-3-*Gen von *C. elegans,* einen Defekt, insbesondere den Eiablagedefekt einer *sel-12-*Mutante unterdrücken können. Diese Mutationen zeichnen sich dadurch aus, dass sie die Expression oder Aktivität von *ses-3* verändern, insbesondere vermindern. Das heißt, bestimmte Mutationen in dem *ses-3*-Gen führen dazu, dass z.B. der Eiablagedefekt, der durch bestimmte *sel-12*-Mutationen verursacht wird, behoben wird und die Doppelmutanten *(sel-12⁻; ses- 3⁻*) wieder einen normalen Wildtyp-Phänotyp zeigen. Genauere Untersuchungen ergaben, daß diese Mutationen in dem *ses-3*-Gen bei den *sel-12*-Mutanten zu einer Aktivierung von *hop-1* führen, d.h. das *hop-1* Presenilin-Protein übernimmt die Funktion des defekten *sel-12* Presenilin-Proteins (Figur 1).

Die gegenwärtigen Erfinder haben das neue ses-3-Gen auf dem Genom von *C*. *elegans* lokalisiert und sequenziert.

Die vorliegende Erfindung offenbart ein isoliertes Nukleinsäuremolekül, das für SES-3 Protein codiert, sowie

Weitere Nukleinsäuremoleküle, die für mutierte SES-3 Proteine codieren.

Darüber hinaus offenbart die Erfindung einen Vektor, der solche isolierten Nukleinsäuremoleküle oder Fragmente davon umfasst.

Weiterhin offenbart die Erfindung die von den Nukleinsäuremolekühlen codierten SES-3 Proteine und mutierte SES-3-Proteine oder Fragmente davon sowie Antikörper, die mit Hilfe dieser Proteine erzeugt wurden.

Somit betrifft die Erfindung einen transgenen C. elegans, welcher ein isoliertes, für SES-3-Protein codierendes Nukleinsäuremolekül oder ein isoliertes, mutiertes Nukleinsäuremolekül umfasst. Insbesondere betrifft die Erfindung transgene C. elegans, die ein ses-3 Allel aufweisen, das die Aktivität von ses-3 vermindert resp. Die Aktivität von sel-12 oder hop-1 steigert. In diesem Zusammenhang wird unter transgenem C. elegans auch ein C. elegans verstanden, bei dem das Genom durch Mutation verändert wurde, und der dadurch ein mutiertes ses-3 Gen aufweist, oder dem das ses-3 Gen entfernt worden ist.

Schließlich betrifft die Erfindung Verwendungen solcher Nukleinsäuremoleküle zur Erzeugung von Modellsystemen zur weiteren Erforschung dieser Erkrankung.

Insbesondere betrifft die Erfindung die Verwendung von transgenen C. elegans in einem Verfahren zur Identifizierung und/oder Charakterisierung von Substanzen, die die Aktivität von ses-3 vermindern resp. Die Aktivität von sel-12 oder hop-1 steigern.

Genau gesagt betrifft die Erfindung transgenen C. elegans, der ein Allel des ses-3 Gens aufweist, das den Eiablagedefekt-Phänotyp (Eg1) in sel-12C. elegans Mutanten kompensiert, wobei das Ses-3 Gen "
a) die Sequenz gemäß SEQ ID NO 2 aufweist, oder
b) für ein Protein mit der Aminosäurensequenz gemäß SEQ ID NO 1 codiert.

Die Erfindung betrifft ferner die Verwendung eines isolierten Nukleinsäuremoleküls mit der Sequenz gemäß SEQ ID NO 2 oder 3 oder eines isolierten Nukleinsäuremoleküls, das für Ses-3 mit der Sequenz gemäß SEQ IS NO 1 kodiert, oder eines isolierten Nukleinsäuremoleküls mit Sequenz 2 oder 3, wobei das letztere Nukleinsäuremolekül eine oder beide der folgenden Mutationen aufweist:
a) Deletion in Exon 8 von Position 2015-2169 in Bezug auf SEQ ID NO 2
b) TC3 Insertion an Position 1817 in Bezug auf SEQ ID NO 2 zur Erzeugung eines Modellorganismus zur Untersuchung und Erforschung neurologischer, insbesondere neurodegenerativer Erkrankungen.

Gemäß einer bevorzugten Ausführungsform der erfindungsgemäßen Verwendung ist das Nukleinsäuremolekül ein DNA-Molekül, insbesondere ein cDNA-Molekül oder ein genomisches DNA Molekül, oder ein RNA-Molekül.

Gemäß einer weiteren bevorzugten Ausführungsform der erfindungsgemäßen Verwendung weist das SES-3 die identische oder im wesentlichen dieselbe Aminosäuresequenz wie die in SEQIDNO: 1 gezeigte Aminosäuresequenz auf, wobei die im wesentlichen selbe Aminosäuresequenz eine Sequenzähnlichkeit aufgrund von Substitution, Deletion, Insertion oder Addition von mindestens 90% zu der in SEQIDNO: 1 gezeigten Aminosäuresequenz aufweist.

### Detaillierte Beschreibung der Erfindung

Die vorliegende Erfindung basiert darauf, dass ein neues Gen, *ses-3,* in *C. elegans* identifiziert werden konnte, welches mit den *C. elegans*-Presenilin-Genen *sel-12* und *hop-1* wechselwirkt und damit eine wichtige Rolle bei der Entwicklung der Alzheimer'schen Krankheit spielt.

So führten verschiedene Mutationen in dem *ses-3*-Gen, darunter auch die vollständige Deletion des *ses-3*-Gens, dazu, dass bestimmte von *sel-12*-Mutationen hervorgerufene veränderte Phänotypen supprimiert bzw. unterdrückt wurden. Die Suppression solcher *sel-12*-Defekte in ses-3-Mutanten wird dadurch erreicht, dass sie die Expression von *hop-1* deutlich dereprimieren und das HOP-1 Protein vermutlich die Funktion des defekten *sel-12-*Proteins übernehmen kann.

Das identifizierte *ses-3*-Gen codiert für ein Protein, das eine FAD Bindestelle zwischen Aminosäure 137 und Aminosäure 264 enthält sowie ein Amino-Oxidase-Motiv zwischen Aminosäure 264 und Aminosäure 677 (Figur 2). Das SES-3 Protein zeigt phylogenetische Verwandtschaft zu dem humanen Protein KIAA0601 und zu dem Drosophila melanogaster Protein ALT1, außerdem wurde durch Computerrecherche (BLAST Suchprogramm) ein Gen für ein weiteres, ähnliches *C. elegans* Protein T08D10.2 identifiziert. Der Grad der Homologie ist in den Figuren 3 und 4 beschrieben.

Somit offenbart die vorliegende Erfindung ein isoliertes Nukleinsäuremolekül, das für SES-3-Protein codiert. Weiterhin offenbart die Erfindung isolierte Nukleinsäuremoleküle, die für mutierte SES-3 Proteine codieren.

Genau gesagt offenbart die Erfindung isolierte Nukleinsäuremoleküle, insbesondere DNA-, wie cDNA- oder genomische DNA-Moleküle, aber auch z.B. RNA-Moleküle, die ein SES-3 Protein aus C. *elegans* mit der in SEQ ID NR:1 angegebenen Aminosäuresequenz codieren, insbesondere jene, die eine in SEQ ID NR:2 angegebene Nukleinsäuresequenz aufweisen.

In der Erfindung ebenfalls beschrieben werden auch isolierte Nukleinsäuremoleküle, deren Nukleotidsequenz mindestens 75%, insbesondere mindestens 80%, speziell mindestens 85%, bevorzugt mindestens 90%, besonders bevorzugt mindestens 95% und am meisten bevorzugt mindestens 98% Sequenzähnlichkeit zu den erwähnten Nukleinsäuremolekülen aufweisen, insbesondere solche, die unter stringenten Bedingungen mit den erwähnten Nukleinsäuremolekülen hybridisieren. Vorzugsweise erfolgt eine solche Hybridisierung unter niedrig stringenten Bedingungen, in einer weiteren Ausführungsform auch bei hochstringenten Bedingungen. Im Rahmen dieser Beschreibung wird unter niedrig stringenten Bedingungen eine Hybridisierung in 3 x SSC bei Raumtemperatur bis 65°C und unter hochstringenten Bedingungen eine Hybridisierung in 0,1 x SSC bei 68°C verstanden. SSC steht als Abkürzung für einen 0,15 M Natriumchlorid, 0,015 M Trinatriumcitrat-Puffer.

Die Nukleinsäuremoleküle mit Nukleinsäuresequenzähnlichkeit zu der in SEQ ID NR:2 angegebenen Nukleinsäuresequenz umfassen insbesondere auch allelische Varianten der angegebenen Nukleinsäuresequenz, die insbesondere die oben erwähnten Mutationen einschließen.

Die Erfindung offenbart auch die Nukleinsäuremoleküle mit jeweils zu den oben erläuterten Nukleinsäuresequenzen komplementärer Nukleinsäuresequenz.

Der Begriff "isoliertes Nukleinsäuremolekül" bezieht sich im vorliegenden Zusammenhang auf Nukleinsäuremoleküle, die im wesentlichen gereinigt von der Hauptmenge der andersartigen Nukleinsäuremoleküle aus den Ausgangs- bzw. Produzentenzellen vorliegen. Präparationen von isolierten Nukleinsäuremolekülen können jedoch ohne weiteres weitere Bestandteile, wie Salze, Substanzen aus dem Medium oder auch restliche Bestandteile der Produzentenzellen, wie z.B. diverse Proteine, enthalten.

Die Erfindung beschreibt ebenso Vektoren, welche ein erfindungsgemäßes Nukleinsäuremolekül, das für SES-3 Protein oder mutierte SES-3 Proteine codiert, enthalten. Beschrieben sind auch Vektoren, die ein Fragment eines erfindungsgemäßen Nukleinsäuremoleküls aufweisen. Das Nukleinsäuremolekül ist dabei mit einem Promoter verknüpft, der für eine Expression des Nukleinsäuremoleküls in dem zur Expression verwendeten Organismus sorgt. Für eine Expression in *C*. *elegans* wird dabei insbesondere einer der bekannten *C. elegans*-Promotoren (http://chinook.uoregon.edu/promoters.html) verwendet.

Der Vektor kann beispielsweise ein Plasmid, ein Cosmid, ein Bacmid, ein Virus oder ein Bakteriophage sein.

Die Erfindung beschreibt auch die Polypeptide oder Proteine, die von den erläuterten Nukleinsäuremolekülen codiert werden, insbesondere SES-3 Protein aus *C. elegans* mit der in SEQ ID NR:1 angegebenen Aminosäuresequenz und die oben erwähnten mutierten SES-3 Proteine sowie davon durch Substitution, Modifizierung, Deletion, Insertion oder Hinzufügung von Aminosäuren abgeleitete Polypeptide oder Proteine, die mindestens 75%, insbesondere mindestens 80%, speziell mindestens 85%, bevorzugt mindestens 90%, besonders bevorzugt mindestens 95% und am meisten bevorzugen mindestens 98% Sequenzähnlichkeit zu den erwähnten Aminosäuresequenzen aufweisen. Gleichenfalls beschrieben sind Fusionsproteine, die Polypeptide, Proteine oder Proteinfragmente fusioniert an ein andersartiges Protein oder einen andersartigen Proteinabschnitt, z.B. ein Marker- oder Indikatorprotein, enthalten. Teil der Erfindung sind auch Fragmente der oben erwähnten Proteine oder Polypeptide.

Die Erfindung erstreckt sich auf transgene Organismen, die eine rekombinante Nukleinsäuresequenz - ggf. in ein Chromosom integriert oder auch extrachromosomal - umfassen, die ein erfindungsgemäßes Nukleinsäuremolekül, das für Ses-3 Protein, mutiertes Ses-3 Protein oder Fragmente davon codiert, als Transgen enthält. Der transgene Organismus ist C. elegans, der Ses-3 Protein aus C. elegans oder ein davon abgeleitetes Polypeptid oder Protein exprimiert.

Gemäß einem Aspekt der Erfindung weisen transgene C. elegans ein ses-3 Allel auf, das die Aktivität von ses-3 vermindert, verstärkt oder ausschaltet. Teil der Erfindung sind auch solche transgene C. elegans, denen das ses-3-Gen vollständig entfernt worden ist. Insbesondere betrifft die vorliegende Erfindung transgene C. elegans, die ein ses-3 Allel aufweisen, das den Eiablagedefekt Phänotyp (Egl) in sel-12-Mutanten kompensiert. Zudem betrifft die vorliegende Erfindung transgene C. elegans, die ein ses-3 Allel aufweisen, das die Aktivität von hop-1 oder sel-12 steigert.

Die Erfindung erfasst auch die Verwendung des vorstehend beschriebenen Nukleinsäuremoleküls zur Erzeugung eines Modellorganismus zur weiteren Untersuchung und Erforschung neurologischer, insbesondere neurodegenerativer Erkrankungen.

Arzneimittel zur Behandlung neurologischer, insbesondere neurodegenerativer Erkrankungen sind beispielsweise dadurch erhältlich, dass mit Hilfe eines solchen Nukleinsäuremoleküls (ein Modellorganismus aufgebaut wird, wobei die mit Hilfe dieses Modellorganismus aufgefundenen Substanzen zu einem Arzneimittel formuliert werden. Für nähere Einzelheiten hierzu wird z.B. auf die internationale Anmeldung PCT/EP01/03214 desselben Anmelders verwiesen.

Insbesondere betrifft die Erfindung die Verwendung von transgenen C. elegans, die ein vorstehend beschriebenes Nukleinsäuremolekül aufweisen, in einem Verfahren zur Identifizierung und/oder Charakterisierung von Substanzen, die die ses-3 Aktivität verändern, insbesondere vermindern oder eliminieren. Des weiteren betrifft die Erfindung die Verwendung von transgenen C. elegans, die ein vorstehend beschriebenes Nukleinsäuremolekül aufweisen, in einem Verfahren zur Identifizierung und/oder Charakterisierung von Substanzen, die die hop-1 oder sel-12 Aktivität steigern.

Zudem betrifft die Erfindung die Verwendung ebensolcher transgener C. elegans in einem Verfahren zur Identifizierung und/oder Charakterisierung von Substanzen, die als Wirkstoffe zur Behandlung und/oder Prävention der Alzheimerschen Krankheit verwendet werden können. Im Hinblick auf die Analogie zu humanen Zellen und im Hintergrund zur gezeigten funktionalen Ähnlichkeit des Notch Signalwegs und der Presenilinfunktion in C. elegans und Mensch wird davon ausgegangen, dass die Fehlfunktion der FAD Presenilinmutanten äquivalent durch Mutation eines homologen oder funktional äquivalenten Gens zu *ses-3* im Mensch kompensiert werden kann. Ein solches homologes Gen ist insbesondere das Gen KIAA0601. Im Rahmen der Erfindung wird daher erwartet, dass ein Defekt in einem humanen *ses-3* Homologen oder funktional äquivalenten Gen zu einer Steigerung der Expression des nicht von der FAD Mutation betroffenen Presenilin-Gens führt.

Die Erfindung beschreibt die Verwendung von Substanzen, die die Expression eines humanen Presenilin steigern, zur Behandlung der Alzheimer'schen Krankheit, sowie diese Substanzen selbst. Insbesondere fallen unter die Substanzen solche, die das humane Presenilingen 1 oder 2 aktivieren und die Expression des humanen Presenilinproteins 1 oder 2 steigern. Zu diesen Substanzen gehören auch jene, die in *C. elegans* zu einer Veränderung der *ses-3* Aktivität führen, und/oder die *sel-12* oder *hop*-1 Presenilin Aktivität steigern.

Analog kann diese Erfindung auch bei der Behandlung der sporadischen, altersabhängigen und nicht an Mutationen gekoppelten Fälle der Alzheimer'schen Erkankung von Nutzen sein. Dabei wird erwartet, dass die Minderung der Expression durch eine Steigerung der Presenilinaktivität die Bildung von Aß4, insbesondere von Aß42, vermindert und dadurch der Ausbruch der Erkrankung zeitlich verzögert wird.

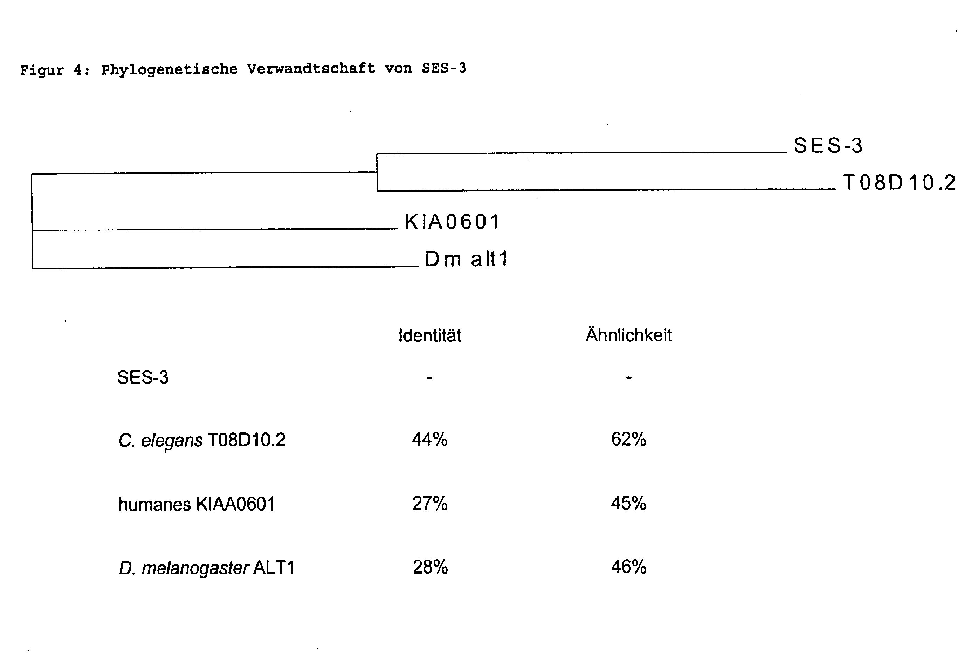

Die Erfindung erwähnt auch die Verwendung des humanen homologen Gens (KIAA0601) sowie die Verwendung des homologen *Drosophila melanogaster* Gens aus Figur 4 (ALT1) sowie funktional ähnlicher Gene, insbesondere aus *C*. *elegans,* zur Auffindung von Substanzen, die als Wirkstoffe zur Behandlung und/oder Prävention der Alzheimer'schen Krankheit verwendet werden können. Insbesondere erwähnt die Erfindung die Verwendung solcher Gene zur Auffindung von Substanzen, die die Aktivität von humanen Presenilinen steigern können. Die Erfindung erwähnt auch transgene Organismen, die diese homologen oder funktional ähnlichen Gene enthalten und die Verwendung solcher Organismen, insbesondere *C*. *elegans* Organismen, in einem Verfahren zur Identifizierung und/oder Charakterisierung von Substanzen, die die *hop-1* oder *sel-12* Aktivität steigern oder die *ses-3* Aktivität vermindern.

Kurze Beschreibung der Figuren und des Sequenzprotokolls
- Fig. 1:: Konzentration von hop-1 in *ses-3* Allelen. Northern Blot, hybridisiert mit einer hop-1 spezifischen Probe.

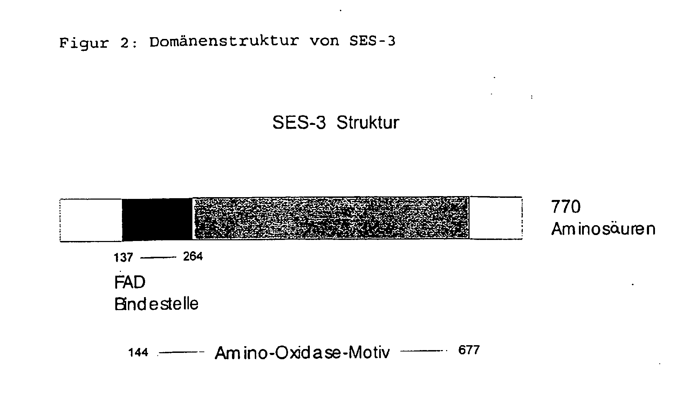

- Fig. 2:: Domänenstruktur von SES-3 Protein.

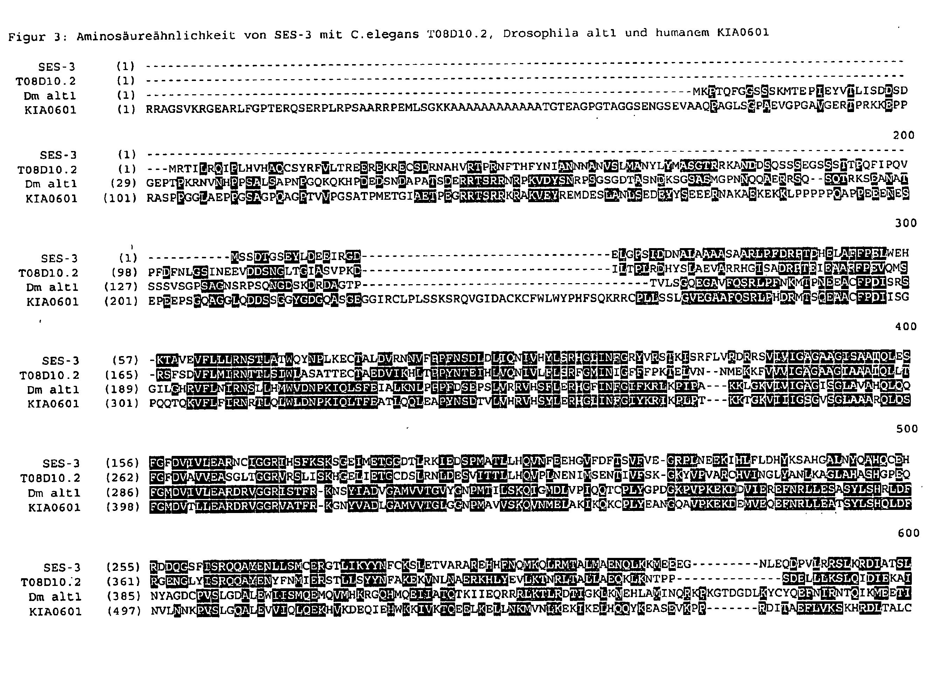

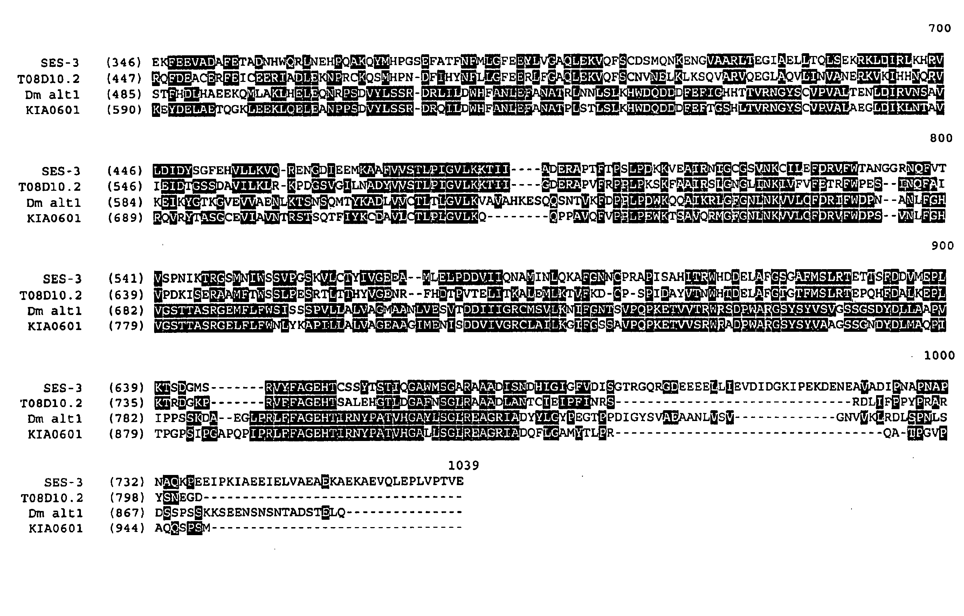

- Fig. 3:: Aminosäurevergleich von SES-3 mit homologen Proteinen.
- Fig. 4:: Phylogenetische Verwandtschaft von SES-3 sowie Homolo- gie.

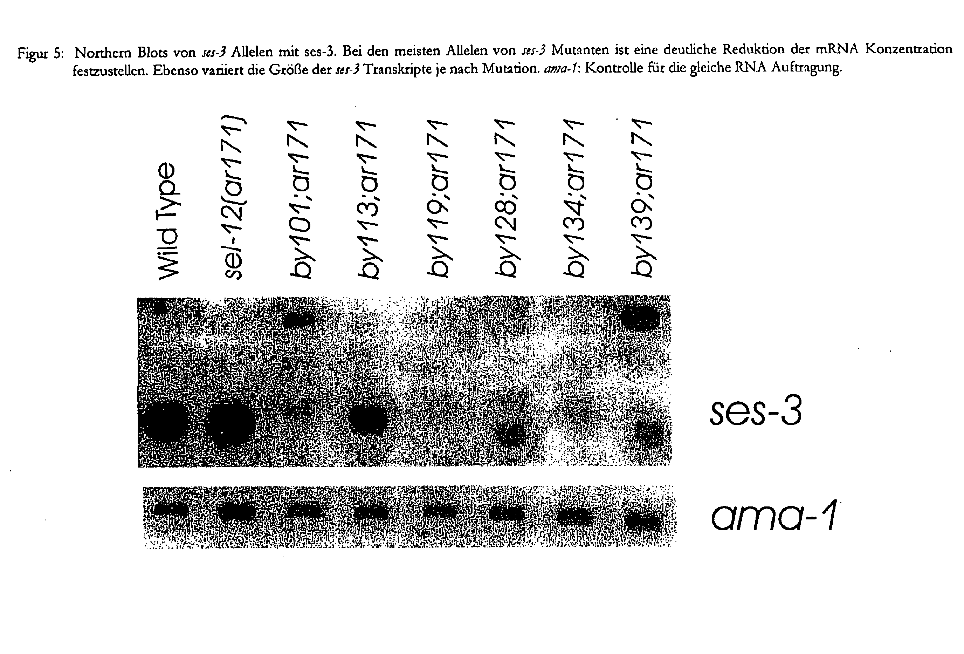

- Fig. 5:: Nachweis der *ses-3* mRNA in Abhängigkeit der verschiede- nen Mutationen. Northern Blot.

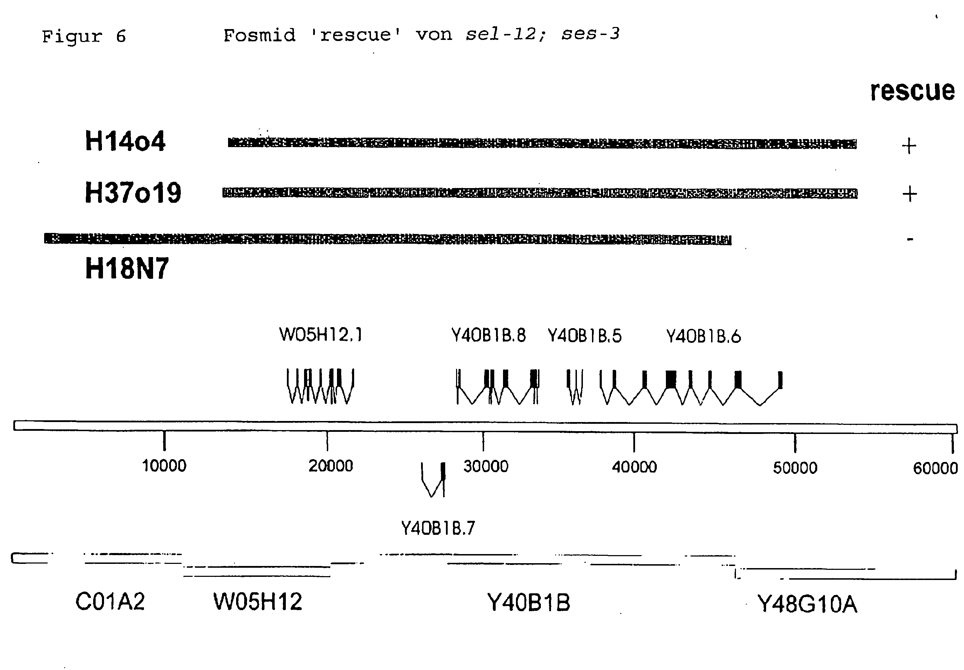

- Fig. 6:: Genomische Struktur der *ses-3* Region und Darstellung der Transgene mit genomischer DNA, die *ses-3* Mutanten komplementieren.

- SEQ ID NR:1:: SES-3 Aminosäuresequenz
- SEQ ID NR:2:: *ses-3* cDNA Sequenz
- SEQ ID NR:3:: Genomische Sequenz eines Subfragments von Y40B1B. 6, das *ses-3* Mutanten komplementieren kann. Dieses Fragment enthält folglich alle für die Ex- pression von *ses-3* notwendigen Sequenzen und Pro- motorabschnitte.

Die Erfindung wird im folgenden anhand von Beispielen näher erläutert.

### Experimenteller Teil

### Isolierung der ses-3 Mutanten

Zur Isolierung von Suppressoren des Eiablagedefektes von sel-12 Mutanten Tieren in *C. elegans* wurde die *sel-12* (ar171). Mutation in einen Mutatorstamm mut-7(pk704) eingekreuzt. Der so erzeugte Stamm zeigt eine temperaturabhängig Aktivierung aller Transposons von *C. elegans* bei 20 °C. Zur Mutagenese wurden *sel-12*(ar171) unc-1(e538); mut-7(pk704) Tiere bei 20 °C auf 3,5 cm NGM Agar Platten mit OP50 kultiviert. Nach jeder Generation wurde nach dem Auftreten von Tieren gesucht, die in der Lage waren, Eier zu legen. Aus der Analyse von etwa 30'000 haploiden Genomen wurden zwei unabhängige Mutanten-Tiere isoliert, die in der Lage waren, reproduzierbar Eier zu legen. Das Eiablageverhalten dieser Tiere war mit dem von Wildtyptieren vergleichbar. Eine dieser Mutationen wurde *ses-3*(by101) genannt.

### Der sel-12; ses-3 Phänotyp

Mutationen in *ses-3* supprimieren den Eiablagedefekt von *sel-12* Mutanten nahezu vollständig. 95% der *sel-12*(ar171); *ses-3*(by101) Doppelmutanten Tiere zeigten ein wildtypähnliches Eiablageverhalten, während 5% der Tiere Defekte in der Eiablage aufwiesen, die den Defekten der *sel-12* Einzelmutante entsprechen. Mutationen in *ses-3* sind in der Lage, alle bekannten *sel-*12 Mutationen zu supprimieren. Daraus kann gefolgert werden, dass *ses-3* nicht allelspezifisch wirkt. *Sel-12*(ar171); ses-3(by101) Doppelmutanten Tiere legen zwischen 180 und 280 Eier, die zu Nachkommen führen; im Gegensatz zur *sel-12*(ar171) Einzelmutante, die im Durchschnitt 60 Nachkommen erzeugt. Die *sel-12*(ar171); *ses-3*(by101) Doppelmutanten Tiere beendenden ihre Eiablage nach etwa 2-3 Tagen und sterben nicht mit einem "bag of worms" Phänotyp, wie für die *sel-12* Einzelmutation üblich. *Sel-12*(ar171); *ses-3*(by101) Doppelmutanten Tiere zeigen gegenüber Wildtyptieren ein um etwa einen Tag verlangsamtes Wachstumsverhalten pro Generation.

### Klonierung des ses-3 Genlocus

Mittels klassischer Genetik wurde *ses-3* als rezessive Mutation auf dem rechten Arm von Chromosom I lokalisiert. Da die *ses-3* Mutation mittels einer Tranposonmutagenese erzeugt wurde, war es wahrscheinlich, dass die Mutation durch die Insertion eines Transposons in den kodierenden Bereich des *ses-3* Genes hervorgerufen wurde. Hierdurch ergibt sich ein s.g. Polymorphismus gegenüber dem *sel-12* Einzelmutanten Stamm. Dieser kann nach Entfernung der restlichen durch den mut-7 Hintergrund erzeugten Polymorphismen mittels Auskreuzung mit dem Wildtyp *C. elegans* Stamm N2. Die Transposons in dem so ausgekreuzten *sel-12*(ar171); *ses-3*(by101) Stamm können mittels Transposondisplay sichtbar gemacht werden. Durch einen Vergleich mit dem Wildtyp sel-12 Stamm können die Transposons identifiziert werden, die nur in den *sel-12*(ar171); *ses-3*(by101) Doppelmutanten Tieren vorhanden sind. Mittels dieses Verfahrens wurde ein Tc3 Insertion im 7 Exon des Gens Y40B1B.6 identifiziert, das durch weiteres Auskreuzen nicht von der *ses-3* Mutation getrennt werden konnte.

### Transgene Rettung des ses-3

Zur Rettung des *ses-3* Phänotyps wurden die.Fosmide H14o4, H37o19 und H18N7 isoliert, die die ses-3 genomische Region beinhalten. Hierbei beinhalten die Fosmide H14o4 und H37o19 die vollständige genomische Sequenz von Y40B1B. 6, während H18N7 nur einen Teil der Y40B1b. 6 genomischen Sequenz beinhaltet, der die Exons 7 und 8 fehlen. Es wurden Transgene mit einzelnen dieser Fosmide in den Stamm *sel-12*(ar171); *ses-3*(by101) injiziert und die Antisuppressor -Aktivität der transgenen Tiere getestet. Alle Klone wurden bei einer Konzentration von 50ng/µl des jeweiligen Fosmids zusammen mit 100ng/µl des dominanten Transformationsmarkers pRF4 (Mello et al., 1991) injiziert. Die Suppression des Eiablagedefektes von sel-12 mutanten Tieren durch ses-3 wurde durch die Anwesenheit der Fosmide H14o4 und H37o19 nahezu vollständig kompensiert (gerettet), während das Fosmid H18N7 nicht in der Lage war, die durch ses-3 vermittelte Suppression von *sel-12* zu retten (siehe Figur 6).

### Struktur von ses-3

*ses-3* (Y40B1B. 6) ist in 8 Exons organisiert und bildet ein Operon mit dem Gen Y40b1B. 5 (siehe Figur 6). Die für Y40B1B. 6 vorhergesagte cDNA stimmt mit der für ses-3 bestimmten cDNA von 2313 Basenpaaren überein (SEQ ID NR:2). *Ses-3* kodiert für ein Protein von 770 Aminosäuren (SEQ ID NR:1). Eine Motivsuche mit Ses-3 ergab, dass Ses-3 eine Amino-Oxidase Motiv über den Grossteil seiner Länge besitzt und am N-Terminus eine FAD Bindestelle (Figur 2). Sequenzvergleiche mit der Datenbank zeigten Homologien zu einem weiteren *C. elegans* Gen T08D10.2, sowie zu einem humanen EST KIAA0601 und einem vorhergesagten Drosophila Gen alt1 (Figur 3). Figur 4 zeigt die verwandtschaftliche Beziehung der Gene untereinander.

### Expression von ses-3

Ein Stadien spezifischer Northern Blot zeigt, dass *ses-3* in allen Entwicklungsstadien exprimiert wird.

### Mutationen in ses-3

by101:
   ist eine Tc3 Insertion im 7 Exon von Y40B1B. 6 an der Position 1817 der cDNA und beeinflusst die mRNA Expression (Figur 5).
by113:
   wurde aus einer EMS Mutagenese isoliert und zeigt keinen Einfluss auf die mRNA Expression (Figur 5).
by119:
   wurde aus einer EMS Mutagenese isoliert und zeigt eine Abschwächung der mRNA Expression (Figur 5).
by128:
   wurde aus einer UV/TMP Mutagenese isoliert und zeigt eine abgeschwächte Expression der mRNA und ein Rearrangement der mRNA und des genomischen Bereichs (Figur 5).
by134:
   wurde aus einer UV/TMP Mutagenese isoliert und zeigt eine abgeschwächte Expression der mRNA (Figur 5).
by139:
   wurde aus einer UV/TMP Mutagenese isoliert und zeigt eine abgeschwächte Expression der mRNA eine verkleinerte Bande, das Auftreten einer zweiten grösseren Bande und eine Deletion in Exon8 von Position 2015 bis 2169 der cDNA (Figur 5).

### Mechanismus der Suppression von sel-12 durch ses-3

*ses-3* Mutationen sind nicht in der Lage, die Sterilität einer *sel-12; hop-1 ses-3* zu supprimieren. Dies demonstriert, dass hop-1 notwendig ist für die Suppression von *sel-12*. Ein Northern Blot der sel-12; ses-3 Doppelmutanten mit den *ses-3* Allelen by101, by119, by128 und by139 zeigt, dass hop-1 Expression in dem L1 Stadium relativ zu *sel-12* und dem Wildtypstamm N2 hochreguliert wird. Da *hop-1* in der Lage ist, *sel-12* zu ersetzen, ersetzt somit *hop-1 sel-12* in den frühen larvalen Stadien.

## Patentansprüche

1. Transgener C. elegans, der ein Allel des SES-3 Gens aufweist, das den Eiablagedefekt-Phänotyp (Egl) in sel-12 C. elegans Mutanten kompensiert, wobei das SES-3 Gen
a) die Sequenz gemäß SEQIDNO 2 aufweist, oder
b) für ein Protein mit der Aminosäuresequenz gemäß SEQIDNO 1 codiert.

2. Transgener C. elegans nach Anspruch 1, der ein SES-3 Allel aufweist, das die Aktivität von hop-1 oder sel-12 steigert.

3. Verwendung eines isolierten Nukleinsäuremoleküls mit der Sequenz gemäß SEQIDNO 2 oder 3 oder eines isolierten Nukleinsäuremoleküls, das für SES-3 mit der Sequenz gemäß SEQIDNO 1 kodiert, oder eines isolierten Nukleinsäuremoleküls mit Sequenz gemäß SEQIDNO 2 oder 3, wobei das letztere Nukleinsäuremolekül eine oder beide der folgenden Mutationen aufweist:
a) Deletion in Exon 8 von Position 2015-2169 in Bezug auf SEQIDNO 2
b) Tc3 Insertion an Position 1817 in Bezug auf SEQIDNO 2 zur Erzeugung eines Modellorganismus zur Untersuchung und Erforschung neurologischer, insbesondere neurodegenerativer Erkrankungen.

4. Verwendung nach Anspruch 3, wobei das Nukleinsäuremolekül ein DNA-Molekül, insbesondere ein cDNA-Molekül oder ein genomisches DNA Molekül, oder ein RNA-Molekül ist.

5. Verwendung nach einem der Ansprüche 3 oder 4, wobei das SES-3 die identische oder im wesentlichen dieselbe Aminosäuresequenz wie die in SEQIDNO: 1 gezeigte Aminosäuresequenz aufweist, wobei die im wesentlichen selbe Aminosäuresequenz eine Sequenzähnlichkeit aufgrund von Substitution, Deletion, Insertion oder Addition von mindestens 90% zu der in SEQIDNO: 1 gezeigten Aminosäuresequenz aufweist.

6. Verwendung nach Anspruch 3, die die in SEQIDNO: 2 angegebene Nukleotidsequenz oder eine dazu komplementäre Sequenz umfasst.

7. Verwendung von transgenem C. elegans nach Anspruch 1 oder 2 in einem Verfahren zur Identifizierung und/oder Charakterisierung von Substanzen, die die SES-3 Aktivität verändern.

8. Verwendung von transgenem C. elegans nach Anspruch 7 in einem Verfahren zur Identifizierung und/oder Charakterisierung von Substanzen, die die SES-3 Aktivität vermindern oder eliminieren.

9. Verwendung von transgenem C. elegans nach Anspruch 1 oder 2 in einem Verfahren zur Identifizierung und/oder Charakterisierung von Substanzen, die die hop-1 oder sel-12 Aktivität steigern.

10. Verwendung von transgenem C. elegans nach Anspruch 1 oder 2 in einem Verfahren zur Identifizierung und/oder Charakterisierung von Substanzen, die als Wirkstoffe zur Behandlung und/oder Prävention der Alzheimer'schen Krankheit verwendet werden können.

## Claims

1. A transgenic C. elegans organism which exhibits an allele of the SES-3 gene which offsets the egg-laying defect phenotype (Egl) in sel-12 C. elegans mutants, wherein the SES-3 gene
a) has the sequence shown in SEQ ID NO 2, or
b) codes for a protein having the amino acid sequence shown in SEQ ID NO 1.

2. A transgenic C. elegans organism as claimed in claim 1 which exhibits an SES-3 allele which increases the activity of hop-1 or sel-12.

3. The use of an isolated nucleic acid molecule having the sequence shown in SEQ ID NO 2 or 3 or of an isolated nucleic acid molecule coding for SES-3 having the sequence shown in SEQ ID NO 1, or of an isolated nucleic acid molecule having the sequence shown in SEQ ID NO 2 or 3, wherein the latter nucleic acid molecule has either or both of the following mutations:
a) deletion in exon 8 from position 2015-2169 in relation to SEQ ID NO 2
b) Tc3 insertion at position 1817 in relation to SEQ ID NO 2
for generating a model organism for investigating and elucidating neurological and more particularly neurodegenerative diseases.

4. The use as claimed in claim 3, wherein the nucleic acid molecule is a DNA molecule, more particularly a cDNA molecule or a genomic DNA molecule, or an RNA molecule.

5. The use as claimed in either of claims 3 and 4, wherein SES-3 has the identical or substantially the same amino acid sequence as the amino acid sequence shown in SEQ ID NO 1, wherein the substantially same amino acid sequence has a sequence similarity owing to a substitution, deletion, insertion or addition of at least 90% to the amino acid sequence shown in SEQ ID NO 1.

6. The use as claimed in claim 3, comprising the nucleotide sequence indicated in SEQ ID NO 2 or a sequence complementary thereto.

7. The use of transgenic C. elegans organisms as claimed in claim 1 or 2 in a method for identifying and/or characterizing substances which alter the activity of SES-3.

8. The use of transgenic C. elegans organisms as claimed in claim 7 in a method for identifying and/or characterizing substances which decrease or eliminate the activity of SES-3.

9. The use of transgenic C. elegans organisms as claimed in claim 1 or 2 in a method for identifying and/or characterizing substances which increase the activity of hop-1 or sel-12.

10. The use of transgenic C. elegans organisms as claimed in claim 1 or 2 in a method for identifying and/or characterizing substances which can be used as active compounds for treating and/or preventing Alzheimer's disease.

## Revendications

1. C. elegans transgénique, qui comprend un allèle du gène SES-3, qui compense le phénotype de défaut de ponte (Egl) dans des mutants sel-12 de C. elegans, où le gène SES-3
a) présente la séquence selon SEQ ID N°2, ou
b) code pour une protéine ayant la séquence d'acides aminés selon SEQ ID N°1.

2. C. elegans transgénique selon la revendication 1, qui comprend un allèle SES-3, qui augmente l'activité de hop-1 et de sel-12.

3. Utilisation d'une molécule d'acide nucléique isolée, ayant la séquence selon SEQ ID N°2 ou 3, ou d'une molécule d'acide nucléique isolée qui code pour le SES-3 ayant la séquence selon SEQ ID N°1, ou d'une molécule d'acide nucléique isolée ayant la séquence selon SEQ ID N°2 ou 3, cette dernière molécule d'acide nucléique comportant l'une des mutations suivantes, ou les dieux :
a) délétion dans l'exon 8, de la position 2015 à la position 2169 par rapport à SEQ ID N°2,
b) insertion Tc3 en position 1817 par rapport à SEQ ID N°2,
pour produire un organisme modèle, destiné à l'étude et à la recherche portant sur des maladies neurologiques, en particulier neurodégénératives.

4. Utilisation selon la revendication 3, pour laquelle la molécule d'acide nucléique est une molécule d'ADN, en particulier une molécule d'ADNc ou une molécule d'ADN génomique ou une molécule d'ARN.

5. Utilisation selon l'une des revendications 3 ou 4, pour laquelle le SES-3 présente une séquence d'acides aminés identique à la séquence d'acides aminés présentée en SEQ ID N°1, ou pour l'essentiel la même que cette dernière, la séquence d'acides aminés qui est pour l'essentiel la même présentant une similitude de séquence, pour ce qui est des substitutions, des délétions, des insertions ou des additions, d'au moins 90 % avec la séquence d'acides aminés présentée en SEQ ID N°1.

6. Utilisation selon la revendication 3, qui comprend la séquence nucléotidique indiquée dans SEQ ID N°2 ou une séquence qui lui est complémentaire.

7. Utilisation de C. elegans transgénique selon la revendication 1 ou 2 dans un procédé destiné à l'identification et/ou à la caractérisation de substances qui modifient l'activité de SES-3.

8. Utilisation de C. elegans transgénique selon la revendication 7 dans un procédé destiné à l'identification et/ou à la caractérisation de substances qui diminuent ou éliminent l'activité de SES-3.

9. Utilisation de C. elegans transgénique selon la revendication 1 ou 2 dans un procédé destiné à l'identification et/ou à la caractérisation de substances qui augmentent l'activité de hop-1 ou de sel-12.

10. Utilisation de C. elegans transgénique selon la revendication 1 ou 2 dans un procédé destiné à l'identification et/ou à la caractérisation de substances qui peuvent être utilisées en tant que principes actifs pour le traitement et/ou la prévention de la maladie d'Alzheimer.


### Sequenzprotokoll

<110> EleGene AG
<120> Identifizierung von *ses-3* sowie dessen Verwendungen
<130> 88 840
<160> 3
<210> 1
   <211> 770
   <212> PRT, Einbuchstabencode
   <213> Caenorhabditit elegans
<223> SES-3 Aminosäuresequenz (Y40B1B. 6)
<400> 1
<210> 2
   <211> 2313
   <212> cDNA
   <213> Caenorhabditit elegans
<223> cDNA Sequenz von ses-3 (Y40B1B. 6)
<400> 2
   atgtcgtctg acacgggttc cgagtatttg gacgaagaga tacgtggcga tgaattgggg ccgtctatag atgataatgc
   tctcgccgct gccgccagcg ccgcccgtct tccattcgac cgcccgactg atcacgagtt ggcattcttc cccgagctct
   gggagcacaa gacagccgtc gaggtgttcc tgctgctcag aaattcgacg ctcgccacgt ggcaatacaa tccactgaaa
   gagtgcacag cgttggatgt cagaaataac gtttttccgc cgttcaactc agatttagac ttgattcaga acattgtgca
   ctatctatcc cgtcatggac taatcaattt cggtcgttac gtgagatcca ccaaaatcag tcgttttcta gtccgtgatc
   ggcgttcagt gattgtaatt ggcgctggag cagctggaat ttcagcagcc acccaactgg aatcattcgg attcgatgta
   atcgttttgg aggcaaggaa ttgtattggt ggacgtattc acagttttaa atcgaaaagt ggagagatta tggagactgg
   aggtgatacg ctgcgaaaaa tcgaggattc accgatggcc acgttgttgc atcaggtaaa cttcgaagag cacggagtat
   tcgatttcac ctcagtattc gtcgagggtc gtccattaaa cgaagaaaag attcatttat tcctagatca ctataaatct
   gctcacggag ccctaaacta tcaagcccat caatgtgagc atcgtgatga tcagggatca tttatttccc gacaacaagc
   ctatgagaat ctattaagta tgtgtgaacg tggaacactt atcaaatatt ataacttttg taaatcactg gaaaccgtcg
   cccgagcccg tgagcatcat ttcaatcaaa tgaaacagtt gagaatgaca gcattgatgg ctgaaaatca gctgaaaaaa
   atggaagaag aaggaaattt ggagcaggat ccggtgctca gaagatcatt aaaacgggat attgcaacat cactcgagaa
   atttgaagaa gtcgctgacg cttttgagac ggctgataat cattggcaac ggcttaatga gcatccacag gcgaaacaat
   atatgcatcc gggatctgaa ttcgcgactt ttaattttat gttgggattt gaggagtatt tggttggagc acaattggaa
   aaagtgcaat tctcgtgtga ttcgatgcaa aataaggaga atggagtggc ggcgagatta accgaaggaa ttgccgaact
   gctaacacaa ttatcggaaa aacgaaaatt ggatattcgt ctgaagcatc gtgttttgga tattgattat agcggatttg
   agcatgtttt actgaaagtt cagcgagaaa atggagatat tgaagaaatg aaagccgctt ttgtagtatc tacacttcca
   attggtgtac ttaaaaaaac aattatcgcc gacgagcgtg ctccgacgtt cactccctca ctacccgata aaaaagtgga
   agcaattcga aatattggct gtggaagtgt gaataagtgt attctcgaat ttgatcgagt tttttggacg gcaaatggtg
   gaagaaatca atttgtgacg gtttcaccga atataaaaac acgtggctcc atgaatatct ggtcatcagt gcctggctca
   aaagtactgt gtacctacat tgtaggcgaa gaggcaatgc tcgagctacc cgatgacgtc ataattcaaa atgcgatgat
   aaatcttcaa aaagcatttg gaaataattg tccacgtgct ccaatcagtg cacatattac acgttggcat gatgatgagt
   tggcatttgg atccggtgca tttatgagct taaggactga aactacaagc tttgatgacg tcatggaacc cctcaaaacc
   tccgatggaa tgtcacgggt ctattttgct ggagaacaca cgtgttccag ctatacatcg acgattcaag gagcatggat
   gtctggggcg agagccgccg ccgacatttc caacgatcat attggcatcg gctttgtgga tataagtgga acacgtggtc
   aacgcggtga tgaagaagaa gagcttctca ttgaagtgga tattgatgga aaaattccgg agaaggatga aaatgaggct
   gtagctgata ttccaaatgc tccaaatgct ccaaatgctc aaaaacccga agaaatcccg aaaatcgccg aagaaattga
   actcgtggct gaagctgaaa aggctgaaaa agctgaagtc cagctggagc cactagtgcc aacagttgaa tag
<210> 3
   <211> 11863
   <212> DNA
   <213> Caenorhabditit elegans
<223> Genomische Sequenz von ses-3 (Y40B1B.6)
<400> 3
   atgtcgtctg acacgggttc cgagtatttg gacgaagaga tacgtggcga tgaattgggg ccgtctatag atgataatgg
   tgaggaaatc ctggaaaaag tgcagaaaat tggataaaaa tggcggtggt agaggctcga gtgtagtttg tagtgtgagc
   ctcaaccaag ttttcagcga aaattcacga ttttcagcag aaaaatcaca gttttgcgct ataataacac caaatataag
   aaaaaaccat tgagttttcc atatttttct gctaaaaaag ctttaaaatt caactttcaa attggttgag gctagaaatg
   ttgtagtttg tagtgtgtga gcctcaacca gtttttcatc catttttaag gcaaaaatag gcaattttac attaaaaatt
   cacgattttc agtagaaaaa tcgcatcttt cgctattaat cgttaaaaac aaaaaaaatt ttctaaagtt ttcgcacaga
   aacaccaaat ttaaaatgaa aatcaatact tggttgaggc tagaaatgtt gtagtttgta gtgtgtgagc ctcaaccaat
   aagttgtgcg atttacattg agaaaatctg aaaagcacag tcaaaaatga gaatttttgg tagaaaacag cattttttcc
   atttaaattc ggaaaaatct cgaaaatgac cttgggtgag gctgagagaa gtgtagtttg tagtctctga gcctcaacca
   atttcttttt tttttcttcg aatttaatat aattttccct gtaatttttt aaattaaagt gtcaaaaatt ccaggaaaaa
   ggtgtagttt gtagtctaat ttccccattt ttttttcagc tctcgccgct gccgccagcg ccgcccgtct tccattcgac
   cgcccgactg atcacgagtt ggcattcttc cccgagctct gggagcacaa gacagccgtc gaggtgttcc tgctgctcag
   aaattcgacg ctcgccacgt ggcaatacaa tccactgaaa gagtgcacag cgttggatgt cagaaataac gtttttccgc
   cgttcaactc agatttagac ttgattcagg tgtggaaatt aggaaaatcg tcgattttgc acctaaaact ttaaaaaaat
   cgagaaaata gatgaaaaac tgcagttttt tgtcgaaaca tccgattttt gtcttgggtg aggctcagac actacaaact
   acaacattta tagcctcaac ctttggttga ggctgaaacg tttgtagttt ttagtctgcg agcctcaacc aaagttgatt
   gtctgatttt tcagatattt catggatttt tcccgaaaaa tacaaatttt taagcaaaaa ttagaatgga aattcccaaa
   aatcggttaa aaacgccaat attcatagag ttcagactac aaactacaaa acaattaaat tttattgaaa aatgaaaaat
   ttaaaatgaa atttcaaaat taaaaggcga aattatatca aaataaccaa aaacattttt gaattttcaa caaaaacctg
   gttttttgaa gaatttcaat ttttttcacc gaaaaatcta attaaaaact acaaaatgaa aaaaaatttg ttttttttgt
   tcgtgaaaaa ttatattcta taaattttaa aattataagt attaaaaaaa taacgttttt ccgccgttca actcggattt
   ggacttgatt caggtgtgaa aattaggatt ttcactgatt ttgcatctaa aaatcatgaa aaactccaga aaatcgatta
   aaaaaccgca gttttcagcg aaaaatgggt gaaaaatccg atttttgtct tgggtgaggc tcagacacta caaactacaa
   tttgcgctga gcctcaacca atcaattttt gctttttttt agaatgaaaa ttggatttta tattttatta ttctgatttg
   agatttaaaa ataaaaattt aagaattttg aattttttta aacagaaaat cggaattaaa tgatcaaatt ttttgaaatt
   ttcgggcgaa aagtgattga gtgctggaaa atagttaaac ttttatcaaa ataactgaaa aatttgaatt ttcaatcatg
   aaaatggcaa attcagggat tttgatgaaa tttttatcga aaaaatcgaa ttttcacctg aaaatttatt gaaaatgctt
   atttttttgc gaattttgac aaaaaaaaac cgaaaaaaat tcctttccga atattttttt acagaaaatc ggaatgaaat
   tttcaaaatt ttgaaatttt cgggcgaaaa gtgattgagt gctggaaagt agttgaaaat tatatttttt ggctattttt
   atagaaattt aaaaaaaagg attattgaaa aaatcgaatt ttctcctgaa aatttattga aaatgcttat ttttttgcga
   attttgacaa aaaaaaaccg aaaaaaattc ctttccgaat atttttttac agaaaatcgg aatgaaagtt tgagaaaatt
   gcgaaataac taacaaaaaa aattgaattt tcaaaaaaaa actggttttt tttttttgaa gaatttcaaa ttttttcact
   gaaaaatcaa ataaaatgtt ttttgggaaa attgcaaaat ttcattttaa aaaaatcgaa ttttcaactg aaagtttatt
   gaaaatccta tttttttgca aaaaatgagg ttttttcaag aatttttgat aaattacttt ccaaattttg tagtttgtag
   tttcaatttt cagaacattg tgcactatct atcccgtcat ggactaatca atttcggtcg ttacgtgaga tccaccaaaa
   tcagtcgttt tctagtccgt gatcggcgtt cagtgattgt aattggcgct ggagcagctg gaatttcagc agccacccaa
   ctggaatcat tcggattcga tgtaatcgtt ttggaggcaa ggaattgtat tggtggacgt attcacagtt ttaaatcgaa
   aagtggagag attatggaga ctggaggtga tacgctgcga aaaatcgagg attcaccgat ggccacgttg ttgcatcagg
   tttgtgcgga aaatcggtgg aaattgcgga aaagtcgagg aaaattgatt aaaaattgga gattttatca aaaatccgat
   ttttgtatca aaaacttgtg aaaaacctat gaaaaactgc agatttcggc gaaaaatacc tgaaaaacgg caaatttgta
   gtagaaatat cattttttgt ttaaaaaaat tgtgaacatc gatcaaaaac ggcatatttc agcgaaaaat accggaaaaa
   cgacggaatt taatcaattt cgttttttgg ttgaggctca cactacaaac tacaatcgtt cttagcctca agcattgttc
   tggttgaggc tgaaacgttc gtagtatgta gtctgggcct caaccaaagt ggattgacca atttttcaga aatttcaata
   aaaaggaaaa cattttccgt aatgtttatc gaattttatt gagaaaattg attaaaaaac tgcagatttt agcaaaaata
   ctcgaaaaat gtatttctcc aaaacatcta tgaaaaaatg caatttatta gcgaaaaaac tcgaaaaacg acaaattttt
   tggcaaaaat tcaatttttg ctgaaaaaaa tttagaaaat tgattgaaaa actgcagatt tcaccgaaaa aatcccgaaa
   aacgacgaaa tttaatcaaa atgacgattt tcgttttggt ggaggctcac actacaaact acaatcgttc ttagcctcaa
   ccattgttct ggttgaggct gaagcgtttg tagtttgtag tctgagcctc aaccaaagtt gatcaacgta ttttccagaa
   atttcatgga attttcttga aaaagacaaa ttttccgtaa ctttattgaa aaaatcgaaa aaaaaacgat ttttgaacga
   aaaatcatgt tttcaaaaaa aattggttga ggctcaggct acaaactaca ctttttaatc ctcaaccagt ataaaagtag
   gttaaacatt gtagtttgta gtctgagttg gaaaatttgc aaattttact caactctaaa aactacagta cctgtaactt
   tgtagtttgt agtcagaaat ttaccactcc gcgtgatata tctttcatta caaactacaa atctaaagta tttgtggttt
   gtagtgagag ctcacactac aaactacaca aaacattcaa ttttttgata tcgaaaacga aaaagtgaaa cattttttat
   taaataagtg ttaaatttga caaaattaat tttttttttc cgactttttt tttggatttt tcaataaaac aaaaatttca
   ggtaaacttc gaagagcacg gagtattcga tttcacctca gtattcgtcg agggtcgtcc attaaacgaa gaaaagattc
   atttattcct agatcactat aaatctgctc acggagccct aaactatcaa gcccatcaat gtgagcatcg tgatgatcag
   ggatcattta tttcccgaca acaagcctat gagaatctat taagtatgtg tgaacgtgga acacttatca aatattataa
   cttttgtaaa tcactggaaa ccgtcgcccg agcccgtgag catcatttca atcaaatgaa acagttgaga atgacagcat
   tgatggctga aaatcagctg aaaaaaatgg aagaagaagg aaatttggag caggatccgg tgctcagaag atcattaaaa
   cgggatattg caacatcact cgagaaattt gaagaagtcg ctgacgcttt tgagacggct gataatcatt ggcaacggct
   taatgagcat ccacaggcga aacaatatat gcatccggga tctgaattcg cgacttttaa ttttatgttg ggatttgagg
   agtatttggt tggagcacaa ttggaaaaag tgcaattctc gtgtgattcg atgcaaaata aggagaatgg agtggcggcg
   agatgttagt gatttgaaaa gagcaaaatt tgatggaaaa tttggaattt tttcgttgaa gaatttgaaa gtttaaggaa
   aatgggaaaa tttaaatttt ggactttttt ttgacaaaaa attggcaaaa ttttataaaa gtgcgaaaaa atcgaaaatt
   ttcgattttt cgaaaaatcg aacgagaaga aaattcaatg gaaaacattt gaaatttcat gaagaaacct gaaaattgtg
   acgaattttc gatttttagg caaaaaaaat gatttcctat tttttttcac tttcgaaaaa aaaactgaaa ttttgtcaaa
   caaacttaat tcaatttttt taaagaattt ttctgccaaa aattggattc tctatttatt ttcgtgttaa cgcatttgaa
   attcgagaaa aaattggaaa ttttcaattt tggaattttt tggcgaaaaa aaattccgca aaaattgata aaattgcaga
   aaaaacggaa atttttagcg aaaaatagct tttcctgaaa attgaaaaat tcacgtgtat tttttgattt tttgaatttt
   tttcctaata aagtcgtaaa aatctgtgaa aattcacgaa aatctttcaa attttcggat aattcagttc aaaacgctcg
   aaaatttgaa taataatcca aaatccacgt ggatttattg attttttgaa tgatttttgg attttggata gaaaatgtca
   aaaatcgtca aaatactaac tagaacagtg ataattgaaa tttttagact atacaggctt aatccaatga aatataatca
   tcaataaaat tacagtaacc gaaggaattg ccgaactgct aacacaatta tcggaaaaac gaaaattgga tattcgtctg
   aagcatcgtg ttttggatat tgattatagc ggatttgagc atgttttact gaaagttcag cgagaaaatg gagatattga
   agaaatgaaa gccgcttttg tagtatctac acttccaatt ggtgtactta aaaagtacgt gccatttttc gagaaaaaaa
   ttgattcgaa aaaaaaacga atttagcgta gtcgttttag aagcactgtt aaaaaaattg cagaaaaaaa ttgaaaaata
   accgaaaatc gcggggtttt ttgccgaaaa cgctgaattt ttcaggaaaa aaacgaaatt ttaaaaaata agtttatgcc
   gaaaaatcag taaaaaattg aatttttaac ttaaattaac gccaaaaact aaaaaaaaaa ccacattttt ccaagaaaat
   tgcgaaattt tcactgaaaa aactcaaaac tagaggaatt cattcaatta ggtgattaaa atagatattt gcctacaaca
   ttctgaaaat tcgaattttt atcgctgaaa aatggggaaa tgcggatttt tccaaaaaaa aacgaaattt tcagagtaaa
   aactagctat ttggctttaa aatttgaaaa attgagtttt tgtttttttt ccttcaaaaa attgagctga aatgaaaaaa
   aacacatttt tccagaaaaa agtagcaaaa ttttcattga aaaaactcaa aaactagaga aattctgtac atttttcaga
   ttaaaatcga tatttggcta caaaattcta aaaaaaaaat tgaattttta tcacaaaaag tacaggaaaa tgtttaaaac
   taagagaact tgaattttat tgtttttttc atctttttaa tcaataaaat gcaagttctc ttaagtttta tacattttcg
   tgtatttttt gtgataaaaa ttcaattttt ttttttagaa ttttgtagcc aaatatcgat tttaatctga aaaatgtaca
   gaatttctct atttttgatt tttttcaatg aaaattttgc tcgtttcgac tcaatttaac tcaaattttc aaattttcca
   aatagctatt ttaagtctaa aaattcgaaa atctccgttt ttcaccggaa aaatccgaaa aaatatgcat ttttaagatc
   aaagatcaat tttttagtag taaaaaattc gactacacca cctttaaata aaaaataaca cattttccag aacaattatc
   gccgacgagc gtgctccgac gttcactccc tcactacccg ataaaaaagt ggaagcaatt cgaaatattg gctgtggaag
   tgtgaataag tgtattctcg aatttgatcg agttttttgg acggcaaatg gtggaagaaa tcaatttgtg acggtttcac
   cgaatatgtg agcggttttt tcggactaca aactataaaa cgtactggtt gaggctcaaa gactacaaac tacaaatttc
   tgaggctcac caagttttct gtcgtttttt aagtttttcc gaacgaattt ttccctgaaa aactaggttt ttgaacaaaa
   tttctgttgt ttttttcggg tttctattcg atttttaaac caaatcttgt cagttcaaat ttgattgagg cgtagactac
   aaactacact ttttctgtta gcctcaacca tttttcgcag aattttgcag tatttttttt tttttgaaaa atcctaattt
   tcgtttaaaa aatcaagatc ttggcccgaa aatagcgaaa aaaacgtcaa atttgtattt taaaagaaca taaattttgg
   ttgaggctca gagactacaa actacaattc atcctcagcc tcaaccaatt actagtgttg aaaatgtcac atttttagtt
   acaacgttgg gttttgaccg ttttctccta tttttcgatg acaaaattta agatttagca agaaaaatct ttaaggaaac
   tgaaattcgc tcgaaaatag caaaaaaatt tcaaattttc gaagaaaagc ttttcactat tttggttgag gctcatagac
   tacaaactac aatttttgtt agcctcaacc atagcgaaat tattggaatt aaaaaaaaat atttcgttaa aaaatttgtt
   tttttaaaga gtgacgaatc acactacaaa ctacacaatt ctaggaaaaa ccgaattttt ttccgataaa aaattttttg
   aaatcaggtt tttgactaca aactacaaaa aaaaaaagtt atttttggct aaaaaatctg aaaaaacact ttttttggta
   ataaaatttt atattttcgg tagtttgtag tgtgtacatt tcctaaaatt ttcgataaaa atcaattttt ttttgaaaaa
   aattttggag ctcccacaaa actacaaact acaaaatgtt aaaatttaat tttgaaaaaa aaaattgtct gatttttaga
   tttgtagttt gtagcctttt gaaaatgaaa tttcctacga atttctcatt aataacgttt attctaagaa gctccgacta
   caaactacaa tattttattt ttaaaacaat tggttgaggc tcagagacta caaactacac aaatctatca gcctcaccca
   taattttaaa ttttcattat atttggatga ggctcagaga ctacaaacta caaactacac aaaattccat tttttctatg
   aagaaatcaa tttttaatag ttttgaaata caaaattgaa aaaaaaaaat tttaattagg ctcccacaaa actacaaact
   acaaaaagtc acattattag atttgtagtt tgcagtctct cattaataac atttattcct ttaaaaaaaa acaagaattg
   tgactgaaaa ataagctccg actacaaact acaatatgac tatttggttg agttgcgcag actacaaact acaaactaca
   caaaattaaa tttttttttc cagaaaaaca cgtggctcca tgaatatctg gtcatcagtg cctggctcaa aagtactgtg
   tacctacatt gtaggcgaag aggcaatgct cgagctaccc gatgacgtca taattcaaaa tgcgatgata aatcttcaaa
   aagcatttgg aaataattgt ccacgtgctc caatcagtgc acatattaca cgttggcatg atgatgagtt ggcatttgga
   tccggtgcat ttatgagctt aaggactgaa actacaagct ttgatgacgt catggaaccc ctcaaaacct ccgatggaat
   gtcacgggtc tattttgctg gagaacacac gtgttccagc tatacatcga cgattcaagg agcatggatg tctggggcga
   ggtacagaca ctacaaacta caaactacac tttttttttt gctggctaca aactacataa ttgtggtgga agcttgtagt
   tgtgaggccc agcgtgacta caaactacac tttttgactg gcgaaaattg caatttctta ctagggaata ttgttaatag
   gggaattgct aggaagacac aacaaacttt aaacgacaga tgaggtcttt ggctacaaaa atcatttttt ggaatctgct
   aaactctgcc ccaaagctga aattttcggc cttaaaaagt agtcctcttt ttgctcaaaa tgccgttttt cgtgtgtaga
   agtggctaca taaaagtttg gcactgcagc cgccgttcga atggccgatt ggtgaactct cgtgctcatt aatctaaagg
   tcacgggttc gttccccact aggtattaat tttttttttt gaatttttcc tctagcatga aaaatgcaca aaaacacaac
   aaattgtcag ttgaatctac tctgctaaat tttatgggta gagagatatg ggcattagta attaagtatg ttaaagcttc
   acttcgatta tttattggta cttttgaaac aactttgcag ttgacaactt ttttttattt tacccgtcaa cggagctgga
   agtctttgaa agtaggtcct tttcgtgaaa aactctagag aaaacaacag tggttttctc ctaaaaatga catttgatcc
   tccaaaatcg tagaaatatc caaactaatg ctgttctttc aaaccaaaca ctttttgaaa aaattttgaa aaattatttt
   tgaagaaaaa tatgtgcata tcaggtggga gaaaagtgaa aatttatcaa aaaaagaaaa cttccaaaaa gtttccaaat
   tacttttatg attaccagga accaaaaaat ttattccgaa gttaccaaaa aacggaagaa accgtgtttt tgtgcatttt
   tcatgctaga ggaaaaattc aaaaaaaaaa ttaataccta gtggggaacg aacccgtgac ctttagatta atgagcacga
   gagttcacca atcggccatt cgaacggcgg ctgcagtgcc aaacttttat gtagccactt ctacacacga aaaacggcat
   tttgagcaaa aagaggacta ctttttaagg ccgaaaattt cagctttggg gcagagttta gcagattcca aaaaatgatt
   tttgtagcca aagacctcat ctgtcgttta aagtttgttg tgtcttccta gcaattcccc tattaacaat attccctagt
   tagcacaaat ccaatttata ttccaacaaa aaaaattgag aaaatcgatg aaaacctgca gatttcagcg aaaagcatcg
   gaaaacgaca aaatttaatc gaaaatccaa tttttgtttt aagtgaggct cacactacaa actacaacat ttttagcctc
   aaccattgtt gtttggttga ggctgaaaat ttgtagtttg tagtccgggc ctcaaccaat gttgattgtc caattttcca
   gaaatttcat ggaattttct tgaaaaagac aaattttctg taactttatt gaaaaaatcg aaaaaaaaaa cgatttttga
   acgaaaaatc acgttttcaa aaaaaatttg gttgaggctc acactacaaa ctacaacatt tttagcctca accattgttg
   tttgattgag gctgaaacgt ttgtagtttg tagtctattg tgaaaatacc tgttttctga aataaaaacg gtgctaaaaa
   gacacgtgga tttattttaa aaaatcgcag ttttctgtag tttgtagtgt aattaagtct cacactacaa actacaatgt
   tttataaaaa ttcggaaaaa aagttattaa aaaatttgaa aattgccttt tttattgaaa taaaaatttt ttacaaaatt
   tccaacaaaa aatgattttc tgtgaaaaaa ttgttcacta caaactacaa aatttcgaga aatcttgtaa aaatttgaaa
   aatgtgataa aattctcata ttttgcttta aagttttttt ttcttacaaa taactttttt tggaaaaaaa tttgaaggtt
   ttcatctttt ttgtagtttg tagtgtaatt agttatacta caaactacaa taatttgtaa cgaattccaa aaatttcgaa
   gaaacatttt tttgttgtag tttgtagtgt aaattcgata atttttacaa tatttccgaa gaaaaagctc taaaaattga
   ggacacacta caaactacaa gataaataaa gttgtagttt gtagtctgag acccttttga aaatatttat aaaaaataca
   gaaagtcacc attttttaac ccaaaattga aaaatctgct atcttaaatt tgttccaaaa aatattgtag tttgtagtct
   acctaaaaaa aactacaaac tacattttct tctatttttc agagccgccg ccgacatttc caacgatcat attggcatcg
   gctttgtgga tataagtgga acacgtggtc aacgcggtga tgaagaagaa gagcttctca ttgaagtgga tattgatgga
   aaaattccgg agaaggatga aaatgaggct gtagctgata ttccaaatgc tccaaatgct ccaaatgctc aaaaacccga
   agaaatcccg aaaatcgccg aagaaattga actcgtggct gaagctgaaa aggctgaaaa agctgaagtc cagctggagc
   cactagtgcc aacagttgaa tag